# EUROPEAN PATENT SPECIFICATION

(11) **EP 4 663 233 B1**
(45) Date of publication and mention of the grant of the patent: **25.11.2026**
(21) Application number: 25182145.0
(22) Date of filing: 11.06.2025
(51) Int. Cl.: A61N 1/365

(54) **SYSTEM FOR PROCESSING HEART SOUND SIGNALS IN AN IMPLANTABLE MEDICAL DEVICE**
SYSTEM ZUR VERARBEITUNG VON HERZTONSIGNALEN IN EINER IMPLANTIERBAREN MEDIZINISCHEN VORRICHTUNG
SYSTÈME DE TRAITEMENT DE SIGNAUX SONORES CARDIAQUES DANS UN DISPOSITIF MÉDICAL IMPLANTABLE

(30) Priority: 14.06.2024 US 202463660170 P; 04.06.2025 US 202519227986
(43) Date of publication of application: 17.12.2025
(73) Proprietor: Pacesetter, Inc., Sylmar, CA 91342 (US)
(72) Inventor: Dawoud, Fady, Sylmar, CA 91342 (US); Gill, Jong, Sylmar, CA 91342 (US)
(74) Representative: Barker Brettell Sweden AB

(56) References cited:
- EP-B1- 3 148 442
- US-A1- 2013 237 872
- US-A1- 2014 005 496
- US-A1- 2020 197 705
- US-B2- 11 051 718

## Description

### TECHNICAL FIELD

Embodiments of the present disclosure relate to methods, devices and systems for processing heart sound signals in an implantable medical device.

### BACKGROUND

Heart sounds can be utilized in a variety of ways to make physiologic determinations about a patient. In particular, heart sounds are created as blood flows through the separate chambers of the heart resulting in four different detectable heart sounds S1, S2, S3, and S4. Conventional approaches that utilize heart sounds may experience certain limitations. For example, various factors may affect the quality of the heart sound signals, such as the location and/or orientation of an implantable medical device (IMD) used to obtain the heart sounds. When the quality of the heart sound signal is inferior, it becomes difficult to identify heart sound characteristics of interest, such as the heart sound duration or peak. When the characteristics of interest are incorrectly detected, the inaccuracy can lead to an incorrect determination of a corresponding interval, such as the interval between S1 and S2 peaks, the interval between the R-wave peak and the S1 peak, etc. Inaccuracies in the interval of interest can lead to incorrect determinations of heart function, diagnoses, treatments, etc.

IMDs equipped with cardiac activity signal measurement such as ECG measurement and also with accelerometer capabilities have been used to collect physiological heart sound (HS) signals. Heart sound bio-signals collected during day-to-day subject activity have less fidelity compared to ECG signals and are more prone to various sources of artifacts (e.g., motion, breathing, electromagnet interference (EMI), etc.) and require more processing to improve signal-to-noise ratio. Thus, a need exists for improved HS signal collection to improve HS data, diagnosis, and resulting treatments.

US 2013/237872 A1 discloses techniques for reducing inappropriate tachyarrhythmia therapy and associated medical device systems. In some examples a processor is enabled to receive a cardiac electrical signal representative of electrical activity of a heart of a patient and provide an indication of cardiac electrical signal reliability. A heart sound analyzing module is enabled to receive the indication of cardiac electrical signal reliability and a heart sound signal representing sounds generated by the heart of the patient and generated by a heart sound sensor. The heart sound analyzing module selectively determines an ensemble averaged heart sound signal or detects a plurality of heart sounds from the heart sound signal in response to the indication of cardiac electrical signal reliability.

EP 3148442 B1 discloses a cardiac rhythm management system senses a cardiac signal indicative of heartbeats and an acoustic signal indicative of heart sounds and detects atrial tachyarrhythmia based on the sensed cardiac and acoustic signals. In various embodiments, the system senses the cardiac and acoustic signals without using an atrial lead, thus allowing for, for example, monitoring atrial fibrillation burden in a heart failure patient who does not wear an implantable device with an atrial lead. In various embodiments, the system detects heartbeats and heart sounds, measures parameters associated with the detected heartbeats and heart sounds, and detects one or more specified types of atrial tachyarrhythmia using the measured parameters. In various embodiments, the measured parameters are selected from heart rate, heart sound amplitude, cycle length variability, and systolic and diastolic intervals.

US 2020/197705 A1 discloses advancing an implantable medical device comprising a tissue-piercing electrode toward the triangle of Koch region of the right atrium or through the coronary sinus to deliver cardiac therapy to or sense electrical activity of the left ventricle in the basal region, septal region, or basalseptal region of the left ventricular myocardium of a patient's heart. The tissue-piercing electrode may include an imageable material. The implantable medical device may include an imageable member made of an imageable material coupled to a housing or a lead of the implantable medical device. Image information representing the patient's heart and the imageable material may be acquired using two-dimensional imaging. Orientation information representing the implantable medical device may be generated based on the acquired image information.

US 11051718 B2 discloses systems and methods for monitoring and treating patients with heart failure are described. A signal receiver may receive a heart sound (HS) signal and an impedance signal sensed from the patient. A heart sound detector circuit may use at least the received impedance signal to determine a HS detection window, and detect a HS component indicative of cardiac diastolic function from the received HS signal within the HS detection window. The system may include a heart failure detector circuit that may generate a cardiac diastolic function indicator (DFI) using the detected HS component and, in certain examples, may detect worsening heart failure using the generated DFI. The system may include a therapy circuit to deliver or adjust an electrostimulation therapy based on DFI.

US 2014/005496 A1 a method for determining the cause of an irregularity in physiologic data collected by a medical device may include monitoring a collected physiologic characteristic of a patient through the physiologic data, detecting an irregularity in the physiologic data, monitoring position data of the patient, correlating the physiologic data with the position data, and determining the cause of the irregularity in the physiologic data based on correlation of the physiologic data with the position data.

### SUMMARY

The invention is defined in the appended claims. Aspects, embodiments and examples disclosed herein which do not fall within the scope of the appended claims do not form part of the invention, and are merely provided for illustrative purposes.

In accordance with embodiments herein, an implantable medical device (IMD) is provided. The IMD comprises a monitoring device configured to obtain cardiac activity signals and an accelerometer configured to obtain candidate heart sound (HS) signals. The IMD also comprises a memory configured to store program instructions and one or more processors configured, when executing the program instructions, to analyze the cardiac activity signals based on an initial criteria, eliminate candidate HS signals based on the analyzing to provide remainder candidate HS signals, analyze the remainder candidate HS signals based on HS quality criteria based on HS characteristics of interest and related to the remainder candidate HS signals, eliminate additional candidate HS signals from the remainder candidate HS signals to provide final HS signals, generate a HS ensemble based on the final HS signals, and control the IMD based on the HS ensemble.

Optionally, the one or more processors are further configured, when executing the program instructions, to determine HS characteristics of interest based on the HS ensemble, analyze the HS characteristics of interest with a dynamic time window, and vary the dynamic time window based on the analyzing of the HS characteristics of interest.

Optionally, the dynamic time window includes a blanking window.

Optionally, the dynamic window includes initial search window bounds that are adjusted based on the HS characteristics of interest.

Optionally, the one or more processors are configured, when executing the program instructions, to analyze a diagnostic characteristic of interest related to the cardiac activity signals.

Optionally, the cardiac activity signals are ECG signals.

Optionally, the diagnostic characteristic of interest is at least one of RR interval, QRS-wave amplitude, R-wave stability, or heart beat rhythm.

Optionally, the initial criteria include at least one of posture or activity level.

Optionally, the HS quality criteria is based on HS characteristics of interest that include at least one of HS amplitude or HS level crossing.

Optionally, the one or more processors are further configured, when executing the program instructions, to determine whether the HS ensemble has a threshold number of beats.

Optionally, the HS quality criteria is a first HS quality criteria. The one or more processors further configured, when executing the program instructions, to analyze the remainder candidate HS signals based a second HS quality criteria, and eliminate the additional candidate HS signals from the remainder candidate HS signals based on the first HS quality criteria and the second HS quality criteria to provide the final group of the final HS signals.

Optionally, the first HS quality criteria is based on HS characteristics of interest that include at least one of HS amplitude or HS level crossing, and the second HS quality criteria is based on HS morphology.

Optionally, the one or more processors are configured, when executing the program instructions, to determine diagnostic characteristics of interest contemporaneously with the candidate HS signals, analyze the diagnostic characteristics of interest based on an initial criteria, determine whether a threshold number of beats are within the HS ensemble and control the IMD based on the HS ensemble when the threshold number of beats are within the HS ensemble.

Optionally, the one or more processors are configured, when executing the program instructions, to determine the diagnostic characteristics of interest is based on the cardiac activity signals.

### BRIEF DESCRIPTION OF THE DRAWINGS

Figure 1 illustrates an exemplary IMD formed in accordance with embodiments herein.
Figure 2 shows a block diagram of an exemplary IMD that is implanted into the patient as part of the implantable cardiac system in accordance with embodiments herein.
Figure 3 illustrates a schematic diagram of a monitoring system in accordance with embodiments herein.
Figure 4 illustrates a schematic block diagram of the external device in accordance with embodiments herein.
Figure 5 illustrates a schematic block flow diagram of a process for obtaining and analyzing a heart sound ensemble.
Figure 6 illustrates a graph of a heart sound ensemble in accordance with embodiments herein.

### DETAILED DESCRIPTION

It will be readily understood that the components of the embodiments as generally described and illustrated in the Figures herein, may be arranged and designed in a wide variety of different configurations in addition to the described example embodiments. Thus, the following more detailed description of the example embodiments, as represented in the Figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all of such operations may be performed by the processor(s) of another device described herein.

### Terms

The term "ensemble" refers to a mathematical combination of two or more data values, signals and the like (e.g., mean, sum, average, median, normalization, etc.). A HS ensemble or HS signal ensemble as used interchangeably herein refer to a mathematical combination of two or more values, signals and the like of heart sounds.

The term "candidate HS signal(s)" refers to any HS signal obtained that may be utilized in a HS ensemble.

The terms "remainder candidate heart sound signals" and "remainder candidate HS signals" as used interchangeably herein refer to any candidate HS signal obtained that may be utilized in a HS ensemble that is not eliminated based on an initial criteria.

The terms "posture" and "patient posture" refer to postural states and/or activity levels of a patient including supine, laying on a right side, laying on a left side, sitting, standing, isometric arm exercises (e.g., pushing, pulling, and the like), ballottement, chest thump, device pressure (e.g., top, mid, and base), arm flap, handshake, and the like.

The term "activity level" refers to intensity and/or types of activity currently experienced by a patient at a point in time, including stationary state, rest state, exercise state, walking state, and the like.

The terms "cardiac activity signal," "cardiac activity signals," "CA signal" and "CA signals" (collectively "CA signals") are used interchangeably throughout to refer to an analog or digital electrical signal recorded by two or more electrodes positioned subcutaneous or cutaneous, where the electrical signals are indicative of cardiac electrical activity. The cardiac activity may be normal/healthy or abnormal/arrhythmic. Non-limiting examples of CA signals include ECG signals collected by cutaneous electrodes, and EGM signals collected by subcutaneous electrodes and/or by electrodes positioned within or proximate to the heart wall and/or chambers of the heart.

The term "heart sound characteristic of interest," "heart sound COI," "HS characteristic of interest," or "HS COI" as interchangeably used herein refers to any signal, measurement, parameter, etc. related to a heart sound. In one example, a heart sound COI is a signal detected by an accelerometer that is proportional to a heart sound. In another example, a heart sound COI is a signal detected by a diaphragm that detects acoustic waves. In yet another example, a heart sound COI includes a signal obtained by a leadless medical device and related to an electrocardiogram. In another example, the heart sound COI is a signal obtained from a haptic sensor that senses vibration corresponding to the heart sounds. In yet another example, the heart sound COI is a signal obtained from a subcutaneous IMD (S-IMD) that includes a pulse generator, and obtains signals related to heart sounds. The signal, measurement, parameter, etc. may be based on a vibration, movement, sound wave, or the like. The heart sound COI may also be detected by other measurement or sensing devices.

The term "contemporaneously" as used herein means to be accomplished at the same time or approximately the same time. For example, if different characteristics of interest are obtained contemporaneously with one another they are considered to be obtained within a five second interval of one another.

The term "diagnostic characteristics of interest" as used herein refers to any signal, measurement, parameter, etc. that are not related to heart sounds or heart sound data. For example, CA signals such as ECG signals may provide measurements, parameters, amplitudes, frequencies, intervals, or the like that are diagnostic characteristics of interest. In another example, posture determinations based on accelerometer signals can be diagnostic characteristics of interest. In addition, information and data from other biological sensors may be utilized to obtain diagnostic characteristics of interest.

The term "initial criteria" when used herein refers to a standard that can be based on measurements, parameters, determinations, etc. that are based on non-HS characteristics of interest (e.g. diagnostic characteristics of interest). The initial criteria are instead based on diagnostic characteristics of interest that are related to other physiological data, connectivity data, or the like that are not related to the HSs. For example, initial criteria can be based on physiological data, information, etc. obtained from ECGs, impedance measurements, accelerometerbased data such as posture, other biosensors, or the like. In examples the initial criteria can include heartrate of the patient during acquisition, static posture such as being supine, having a left-sided recline, having a right, or the like.

The term "quality criteria" when used herein refers to a standard that can be based on measurements, parameters, determinations, etc. that are based on the HS characteristics of interest. For example, quality criteria can include HS COls such as HS amplitude, HS level crossing, or the like.

Embodiments may be implemented in connection with one or more implantable medical devices (IMDs). Non-limiting examples of IMDs include one or more implantable lead-based or leadless therapy devices. For example, the IMD may represent a pacemaker, cardioverter, cardiac rhythm management device, defibrillator, whether lead-based or leadless. For example, the IMD may include one or more structural and/or functional aspects of the device(s) described in U.S. Patent 9,216,285 "Leadless Implantable Medical Device Having Removable And Fixed Components"; U.S. Patent 8,442,634 "Systems and Methods for Controlling Ventricular Pacing in Patients with Long Inter-Atrial Conduction Delays"; and/or U.S. Patent 8,923,965 "Systems and Methods for Optimizing AV/VV Pacing Delays Using Combined IEGM/Impedance-Based Techniques for use with Implantable Medical Devices"; U.S. Patent Application Publication 2014/0039333 "Systems and Methods for Detecting Mechanical Dyssynchrony and Stroke Volume for use with an Implantable Medical Device Employing a Multi-Pole Left Ventricular Lead". Additionally or alternatively, the IMD may include one or more structural and/or functional aspects of the device(s) described in U.S. Patent 8,391,980 "Method And System For Identifying A Potential Lead Failure In An Implantable Medical Device" and U.S. Patent 9,232,485 "System And Method For Selectively Communicating With An Implantable Medical Device".

A system is provided that obtains and analyzes CA signals, including ECG signals and also obtains and analyzes HS signals to improve the quality of the resultant beat ensemble representation of the heart sound. An initial or entry criteria is provided to initiate collection of the heart sound. This set of criteria is based on a sensed CA signal (e.g., ECG signal), posture, activity, activity level, or the like. For example, the criteria for the CA signal may relate to the amplitude and stability of RR intervals, R-wave amplitude and stability and beat rhythm, or the like. The posture criteria as determined utilizing signals from an accelerometer can include mode and stability. Similarly, activity criteria can be based on accelerometer signals, other biosensor signals, a combination of accelerometer signals and biosensor signals, etc. and include level and stability. In yet another example the time of day can be an initial criteria. In particular, the time of day can be used to identify when a user is sleeping. Once the initial criteria are considered and HS signals reduced based on this initial criteria, a subsequent set of beat-by-beat quality criteria can then be utilized to sub-select beats judged to have adequate fidelity to contribute to an ensemble HS waveform for analysis. This set of quality criteria can be based on HS COIs such as HS amplitude, HS level crossing, or the like. Then, using this selected ensemble, dynamic time windows can be used to measure relevant HS COls (e.g., amplitude, integral and timing of S1, S2, S3, S4, or the like) for trending and additional predictive analytics.

In another example, an additional analysis can be undertaken utilizing a second beat-by-beat quality criteria prior to finalizing the ensemble for additional analysis. In such as example the second quality criteria may be based on morphology criteria. The ensemble HS beat representation may be compared to an individual HS morphology on a beat-by-beat basis and if the morphological comparison results in acceptable similarities, then the ensemble HS beat representation is determined to be acceptable. Exemplary comparison method between ensemble HS beat representation and individual HS beats could be any kind of morphology similarity test, including correlation coefficient.

In addition to obtaining, analyzing, and selecting the HS signals that make up the HS ensemble used for analysis, provided is a unique manner of analyzing the selected HS ensemble. In particular, an adaptive process is provided to identify search windows of S1, S2, S3, and S4 components of the ensemble HS waveform. Initial bounds are defined for S1, S2, S3, and S4 assuming a heart rate of 60 bpm and a series of S1 windows with accompanying blanking windows are provided. The blanking windows provide a determined period of time after a first peak (e.g., S1 peak) is identified to prevent an incorrect reading of a different sound peak (e.g., S2 peak when the initial peak is the S1 peak). The initial search window bounds can then be adjusted for heart rate scaling the bounds by a median RR interval. In this manner, the initial search window bounds are based on median heart rate of 60 bpm (or RR interval of 1 sec). Multiplying by RR interval, the search window bounds are scaled to be narrower for faster heart rates (shorter RR interval) or wider for slower heart rates (longer RR interval).

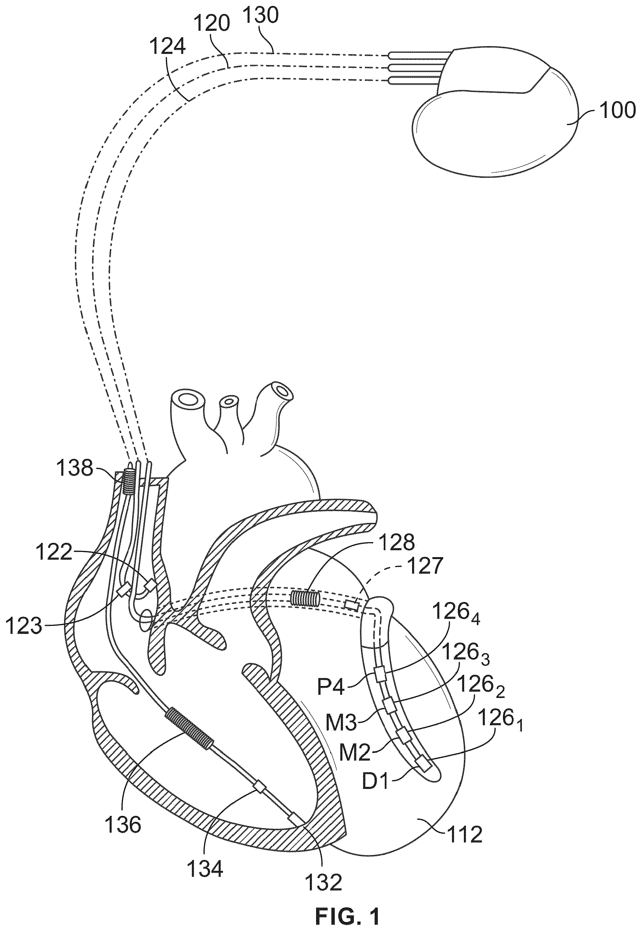

Figure 1 illustrates an exemplary IMD 100 formed in accordance with embodiments herein. The IMD 100 is shown in electrical communication with a heart 112 by way of a right atrial lead 120 having an atrial tip electrode 122 and an atrial ring electrode 123 implanted in the atrial appendage. The IMD 100 is also in electrical communication with the heart by way of a right ventricular lead 130 having, in this embodiment, a ventricular tip electrode 132, a right ventricular ring electrode 134, a right ventricular (RV) coil electrode 136, and a superior vena cava (SVC) coil electrode 138. Typically, the right ventricular lead 130 is transvenously inserted into the heart so as to place the RV coil electrode 136 in the right ventricular apex, and the SVC coil electrode 138 in the superior vena cava. Accordingly, the right ventricular lead is capable of receiving cardiac signals and delivering stimulation in the form of pacing and shock therapy to the right ventricle.

To sense left atrial and ventricular cardiac signals and to provide left chamber pacing therapy, IMD 100 is coupled to a multi-pole LV lead 124 designed for placement in the CS region via the CS OS for positioning a distal electrode adjacent to the left ventricle and/or additional electrode(s) adjacent to the left atrium. An exemplary LV lead 124 is designed to receive atrial and ventricular cardiac signals and to deliver left ventricular pacing therapy using a set of four left ventricular electrodes 126₁, 126₂, 126₃, and 126₄ (thereby providing a quadripole lead), left atrial pacing therapy using at least a left atrial ring electrode 127, and shocking therapy using at least a left atrial coil electrode 128 implanted on or near the left atrium.

While illustrated as a cardioverter defibrillator with multiple transvenous leads inside the heart, in other example embodiments the IMD can be a leadless device. Optionally, the leadless device can include a housing, multiple electrodes coupled to the housing, and a pulse generator hermetically contained within the housing and electrically coupled to the electrodes. A pulse generator may be provided and configured for sourcing energy internal to the housing, generating, and delivering electrical pulses to the electrodes. A controller can also be hermetically contained within the housing as part of the pulse generator and communicatively coupled to the electrodes. The controller can control, among other things, recording of physiologic characteristics of interest and/or electrical pulse delivery based on the sensed activity.

Optionally, a first leadless device can be located in the right atrium (RA), while a second leadless device is located in the right ventricle (RV). The leadless devices coordinate the operation therebetween based in part on information conveyed between the leadless devices during operation. The information conveyed between the leadless devices may include, among other things, physiologic data regarding activity occurring in the corresponding local chamber. For example, the atrial leadless device may perform sensing, including for heart sounds S1, S2, S3, or S4, and pacing operations in the right atrium, while the ventricular leadless device may perform sensing, including heart sound sensing, and pacing operations in the right ventricle.

Alternatively, leadless devices can be located in the RV or left ventricle (LV) to obtain physiologic data regarding atrial activity, including heart sounds S1, S2, S3, or S4. In addition, optionally, the leadless device could be located in the RV or LV to obtain physiologic data regarding activity in one of the LV or RV in order to determine and set a VV delay.

Alternatively, the leadless devices may be located in other chamber combinations of the heart, as well as outside of the heart. Optionally, the leadless devices may be located in a blood pool without directly engaging local tissue. Optionally, the leadless devices may be implemented solely to perform monitoring operations, without delivery of therapy. As another example, one or more leadless devices may represent a subcutaneous implantable device located in a subcutaneous pocket and configured to perform monitoring and/or deliver therapy.

Optionally, the leadless devices include electrodes that are located directly on the housing of the device, without a lead extending from the device housing. Alternatively, the leadless device may be implemented with leads, where the conducted communication occurs between one or more electrodes on the lead and/or on the housing. Examples of other IMDs that may be configured to implement the conducted communication embodiments described herein are described in U.S. patent 9,168,383, issued October 27, 2015, and titled "LEADLESS CARDIAC PACEMAKER WITH CONDUCTED COMMUNICATION".

In particular embodiments, the IMD can be a subcutaneous IMD (S-IMD) that includes a pulse generator that is positioned within a pectoral region of a chest of a patient. Embodiments can also include a lead having first and second electrode segments with the first electrode segment positioned along an anterior of the chest of the patient and the second electrode segment positioned along a posterior of the patient. The first and second electrode segment may obtain physiologic data regarding cardiac activity, including heart sounds S1, S2, S3, or S4.

### Implantable Medical Device and Accelerometer

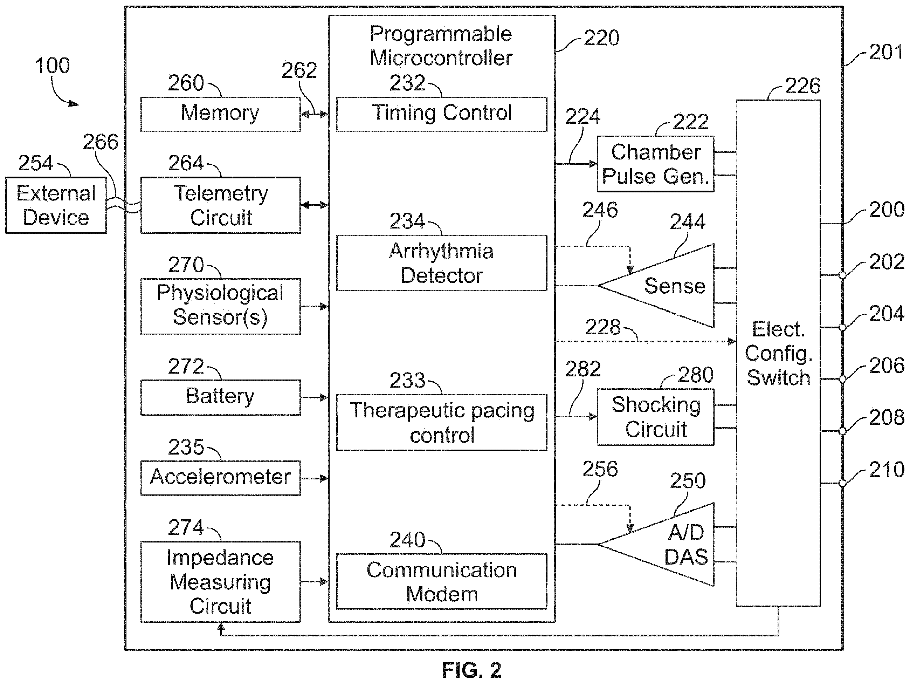

Figure 2 shows a block diagram of an exemplary IMD 100 that is implanted into the patient as part of the implantable cardiac system. The IMD 100 may be implemented as a full-function biventricular pacemaker, equipped with both atrial and ventricular sensing, and pacing circuitry for four chamber sensing and stimulation therapy (including both pacing and shock treatment). Optionally, the IMD 100 may provide full-function cardiac resynchronization therapy. Alternatively, the IMD 100 may be implemented with a reduced set of functions and components. For instance, the IMD may be implemented without ventricular sensing and pacing. As described herein, the IMD 100 is configured to provide LUV pacing therapy without pacing the RV.

The IMD 100 has a housing 201 to hold the electronic/computing components. The housing 201 (which is often referred to as the "can", "case", "encasing", or "case electrode") may be programmably selected to act as the return electrode for certain stimulus modes. Housing 201 further includes a connector (not shown) with a plurality of terminals, a portion of which are designated as terminals 202, 204, 206, 208, and 210. The terminals may be connected to electrodes that are located in various locations within and about the heart. For example, the terminals may include: a terminal 202 to be coupled to an first electrode (e.g., a tip electrode) located in a first chamber; a terminal 204 to be coupled to a second electrode (e.g., tip electrode) located in a second chamber; a terminal 206 to be coupled to an electrode (e.g., ring) located in the first chamber; a terminal 208 to be coupled to an electrode located (e.g., ring electrode) in the second chamber; and a terminal 210 to be coupled to an electrode (e.g., coil) located in the SVC. The type and location of each electrode may vary. For example, the electrodes may include various combinations of ring, tip, coil and shocking electrodes and the like. It is understood that more or fewer terminals may be utilized. With reference to Figure 1, the housing 201 includes at least a number of terminals corresponding to the number of electrodes provided on leads 120, 124 and 130. For example, terminals are provided to connect to the LV electrodes 126₁-126₄.

The IMD 100 includes a programmable microcontroller 220 that controls various operations of the IMD 100, including cardiac monitoring and stimulation therapy. Microcontroller 220 includes a microprocessor (or equivalent control circuitry), RAM and/or ROM memory, logic and timing circuitry, state machine circuitry, and I/O circuitry.

The IMD 100 further includes one or more pulse generators 222 that generates stimulation pulses for delivery by one or more electrodes coupled thereto. The pulse generator 222 is controlled by the microcontroller 220 via control signal 224. The pulse generator 222 is coupled to the select electrode(s) via an electrode configuration switch 226, which includes multiple switches for connecting the desired electrodes to the appropriate I/O circuits, thereby facilitating electrode programmability. The switch 226 is controlled by a control signal 228 from the microcontroller 220.

Microcontroller 220 is illustrated to include timing control circuitry 232 or a timer that is configured to determine timing operations associated with the IMD 100. For example, the timing control circuitry 232 can be provided such that once a determined period of time has passed, different sensors, including biological sensors, accelerometers, detectors, or the like can actuate to obtain data and information related to the IMD, patient, heart of the patient, etc.

Microcontroller 220 also has an arrhythmia detector 234 for detecting arrhythmia conditions and a morphology detector to review and analyze one or more features of the morphology of cardiac signals. In one example the morphology detector can be utilized to determine morphology criteria, determine if morphology criteria are met, or the like.

The microcontroller 220 includes therapeutic pacing control circuitry 233 that is utilized with an accelerometer 235 to implement the processes described herein for controlling a pacing therapy. The control circuitry 233 can be operated based on signals received from the accelerometer 235 that also measures heart sound characteristic of interest (COI) in connection with delivering a pacing therapy by the IMD. A heart sound COI in one example is received from the accelerometer 235. The accelerometer 235 can sense a heart sound COI from any heart sound including S1, S2, S3, or S4. The heart sound COI can be based on a combination of heart sounds, including the interval between S1 and S2, between S2 and S3, between S3 and S4, any other combination or permutation, or the like. In yet another example, the heart sound COI can be a duration of any one heart sound, combination of heart sounds, etc.

In one example, the accelerometer can be implemented utilizing all or portions of the structural and/or functional aspects of the methods and systems described in US Patent 6,937,900, titled "AC/DC Multi-Axis Accelerometer for Determining A Patient Activity and Body Position;" U.S. Patent 11,980,472, titled "SYSTEM FOR VERIFYING A PATHOLOGIC EPISODE USING AN ACCELEROMETER"; U.S. Publication 2021/0345891, filed 5/8/2020, titled "METHOD AND DEVICE FOR DETECTING RESPIRATION ANOMALY FROM LOW FREQUENCY COMPONENT OF ELECTRICAL CARDIAC ACTIVITY SIGNALS;" U.S. Patent Publication 2021/0350931, filed 3/8/2021, titled "METHOD AND SYSTEMS FOR HEART CONDITION DETECTION USING AN ACCELEROMETER".

The IMD 100 is further equipped with a communication modem (modulator/demodulator) 240 to enable wireless communication with other devices, implanted devices, and/or external devices. The communication modem 240 may be implemented in hardware as part of the microcontroller 220, or as software/firmware instructions programmed into and executed by the microcontroller 220. Alternatively, the modem 240 may reside separately from the microcontroller as a standalone component.

The IMD 100 includes sensing circuit 244 selectively coupled to one or more electrodes that perform sensing operations, through the switch 226 to detect the presence of cardiac activity in the right chambers of the heart. The sensing circuit 244 may include dedicated sense amplifiers, multiplexed amplifiers, or shared amplifiers. The output of the sensing circuit 244 is connected to the microcontroller 220 which, in turn, triggers or inhibits the pulse generator 222 in response to the absence or presence of cardiac activity. The sensing circuit 244 receives a control signal 246 from the microcontroller 220 for purposes of controlling the gain, threshold, polarization charge removal circuitry (not shown), and the timing of any blocking circuitry (not shown) coupled to the inputs of the sensing circuit.

The IMD 100 further includes an analog-to-digital (A/D) data acquisition system (DAS) 250 coupled to one or more electrodes via the switch 226 to sample cardiac signals across any pair of desired electrodes. The data acquisition system 250 is configured to acquire intracardiac electrogram signals, convert the raw analog data into digital data, and store the digital data for later processing and/or telemetric transmission to an external device 254 (e.g., a programmer, local transceiver, or a diagnostic system analyzer). The data acquisition system 250 is controlled by a control signal 256 from the microcontroller 220.

The microcontroller 220 is coupled to a memory 260 by a suitable data/address bus 262. The memory 260 may be configured to include patient data, including whether a patient has LBBB. In particular, the control circuitry can determine if a patient has LBBB or can obtain the patient data from the memory.

The operating parameters of the IMD 100 may be non-invasively programmed into the memory 260 through a telemetry circuit 264 in telemetric communication via communication link 266 with the external device 254. The telemetry circuit 264 allows intracardiac electrograms and status information relating to the operation of the IMD 100 (as contained in the microcontroller 220 or memory 260) to be sent to the external device 254 through the established communication link 266.

The IMD 100 can further include one or more physiologic sensors 270. Such sensors are commonly referred to as "rate-responsive" sensors because they are typically used to adjust pacing stimulation rates according to the exercise state of the patient. However, the physiological sensor 270 may further be used to detect changes in cardiac output, changes in the physiological condition of the heart, or diurnal changes in activity. Signals generated by the physiological sensors 270 are passed to the microcontroller 220 for analysis. The microcontroller 220 responds by adjusting the various pacing parameters at which the atrial and ventricular pacing pulses are administered.

A battery 272 provides operating power to all of the components in the IMD 100. The battery 272 is capable of operating at low current drains for extended periods of time and is capable of providing high-current pulses (for capacitor charging) when the patient requires a shock pulse (e.g., in excess of 2 A, at voltages above 2 V, for periods of 10 seconds or more). The IMD 100 further includes an impedance measuring circuit 274 that is enabled by the microcontroller 220 via a control signal 282.

The IMD 100 can be operated as an implantable cardioverter/defibrillator (ICD) device, which detects the occurrence of an arrhythmia and automatically applies an appropriate electrical shock therapy to the heart aimed at terminating the detected arrhythmia. The microcontroller 220 further controls a shocking circuit 280 by way of a control signal 282.

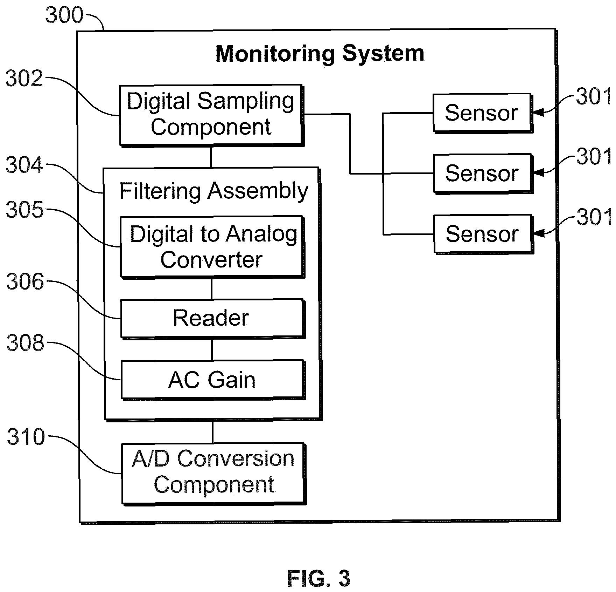

Figure 3 illustrates a schematic diagram of a monitoring system 300 that may be used to detect and determine heart sounds, including S1 sound intervals, and S2 sound intervals. In one example, the monitoring system is or includes an accelerometer. In one embodiment when the monitoring system 300 is a three-dimensional accelerometer, the three-dimensional accelerometer may be a chip for placement in an IMD. In another embodiment, the accelerometer is formed and operates in the manner described in US patent 6,937,900, titled "AC/DC Multi-Axis Accelerometer For Determining A Patient Activity And Body Position". In yet another embodiment, the accelerometer is formed and operates in the manner described in US Patent Publication 2021/0350931, titled Method and System for Heart Condition Detection Using an Accelerometer. In an embodiment, when the monitoring system is an accelerometer, the accelerometer includes sensors that generate first (X), second (Y) and third (Z) accelerometer signals along corresponding X, Y and Z axes (also referred to as first axis accelerometer signals, second axis accelerometer signals and third axis accelerometer signals). The X, Y and Z axes accelerometer signals collectively define a three-dimensional, or multidimensional (MD) accelerometer data set. While examples herein are described in connection with an accelerometer that generates accelerometer signals along three orthogonal axes, it is recognized that embodiments may be implemented wherein accelerometer signals are generated along two or more axes, including more than three axes.

The monitoring system 300 may include sensors 301 that monitor and receive signals from the X, Y and Z axes. In one embodiment, the individual X, Y and Z signals are received by a digital sampling component 302 that receives a digital input. Coupled to the digital sampling component 302 is a filtering assembly 304 that may include a digital to analog converter 305 to form an alternating current (AC) signal, a reader device 306, and an AC gain device 308. While in this embodiment, the filtering assembly includes the devices provided, in other examples, other devices may be utilized to filter the digital input signal for processing.

The monitoring system 300 may also include an analog to digital conversion component 310, along with a position, or direct current (DC) component. In one example, the analog to digital conversion component may be an 8-bit analog to digital converter (ADC). The evaluation version of the monitoring system 100 may provide 3-axis (X and Y along the chip, Z normal to the chip) DC-coupled posture signal corresponding to 3 orthogonal directions as well as 3-axis AC-coupled activity signal. In one embodiment, each of the 6 signal may be sampled at 100 Hz and accumulated over 1 sec for a total of 12 signals([X/Y/Z], [posture/activity], [100/1 Hz]). This MD accelerometer data may be used to describe embodiments herein.

While described as a digital signal in relation to Figure 3, in other embodiments the signal may be an analog signal, filtered, amplified, etc. The accelerometer data signals may be recorded in a data storage of the accelerometer, of an IMD, of a remote device etc. Alternatively, the accelerometer data set may be obtained from a remote device or received from a storage device coupled to the accelerometer. The accelerometer data set may be a multidimensional accelerometer data set.

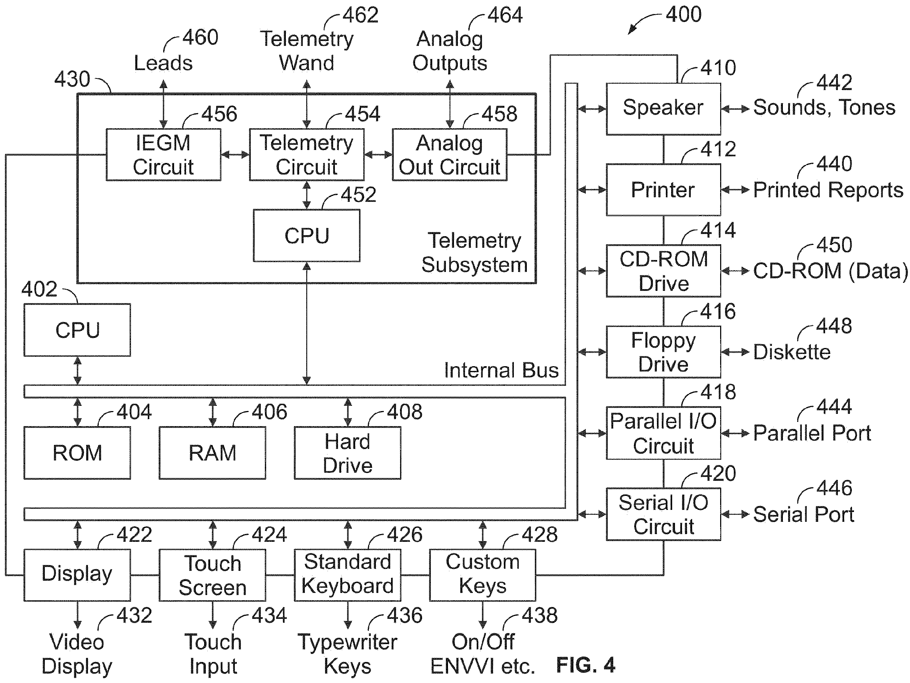

Figure 4 illustrates a functional block diagram of the external device 400 that is operated in accordance with the processes described herein and to interface with implantable medical devices as described herein. The external device 400 may be a workstation, a portable computer, an IMD programmer, a PDA, a cell phone, and the like. The external device 400 includes an internal bus that connects/interfaces with a Central Processing Unit (CPU) 402, ROM 404, RAM 406, a hard drive 408, the speaker 410, a printer 412, a CD-ROM drive 414, a floppy drive 416, a parallel I/O circuit 418, a serial I/O circuit 420, the display 422, a touch screen 424, a standard keyboard connection 426, custom keys 428, and a telemetry subsystem 430. The internal bus is an address/data bus that transfers information between the various components described herein. The hard drive 408 may store operational programs as well as data, such as waveform templates and detection thresholds.

The CPU 402 typically includes a microprocessor, a micro-controller, or equivalent control circuitry, designed specifically to control interfacing with the external device 400 and with the IMD 100. The CPU 402 may include RAM or ROM memory, logic and timing circuitry, state machine circuitry, and I/O circuitry to interface with the IMD 100. The CPU 402 may implement some or all of the operations of the control circuitry 233 (Figure 2). The CPU 402 may implement some or all of the operations of the methods described herein.

The display 422 (e.g., may be connected to the video display 432). The touch screen 424 may display graphic information relating to the IMD 100. The display 422 displays various information related to the processes described herein. The touch screen 424 accepts a user's touch input 434 when selections are made. The keyboard 426 (e.g., a typewriter keyboard 436) allows the user to enter data to the displayed fields, as well as interface with the telemetry subsystem 430. Furthermore, custom keys 428 turn on/off 438 (e.g., EVVI) the external device 400. The printer 412 prints copies of reports 440 for a physician to review or to be placed in a patient file, and speaker 410 provides an audible warning (e.g., sounds and tones 442) to the user. The parallel I/O circuit 418 interfaces with a parallel port 444. The serial I/O circuit 420 interfaces with a serial port 446. The floppy drive 416 accepts diskettes 448. Optionally, the floppy drive 416 may include a USB port or other interface capable of communicating with a USB device such as a memory stick. The CD-ROM drive 414 accepts CD ROMs 450.

The telemetry subsystem 430 includes a central processing unit (CPU) 452 in electrical communication with a telemetry circuit 454, which communicates with both an IEGM circuit 456 and an analog out circuit 458. The circuit 456 may be connected to leads 460. The circuit 456 is also connected to the implantable leads to receive and process IEGM cardiac signals as discussed above. Optionally, the IEGM cardiac signals sensed by the leads may be collected by the IMD 100 and then transmitted to the external device 400, wirelessly to the telemetry subsystem 430 input.

The telemetry circuit 454 is connected to a telemetry wand 462. The analog out circuit 458 includes communication circuits to communicate with analog outputs 464. The external device 400 may wirelessly communicate with the IMD 100 and utilize protocols, such as Bluetooth, GSM, infrared wireless LANs, HIPERLAN, 3G, satellite, as well as circuit and packet data protocols, and the like. Alternatively, a hard-wired connection may be used to connect the external device 400 to the IMD 100.

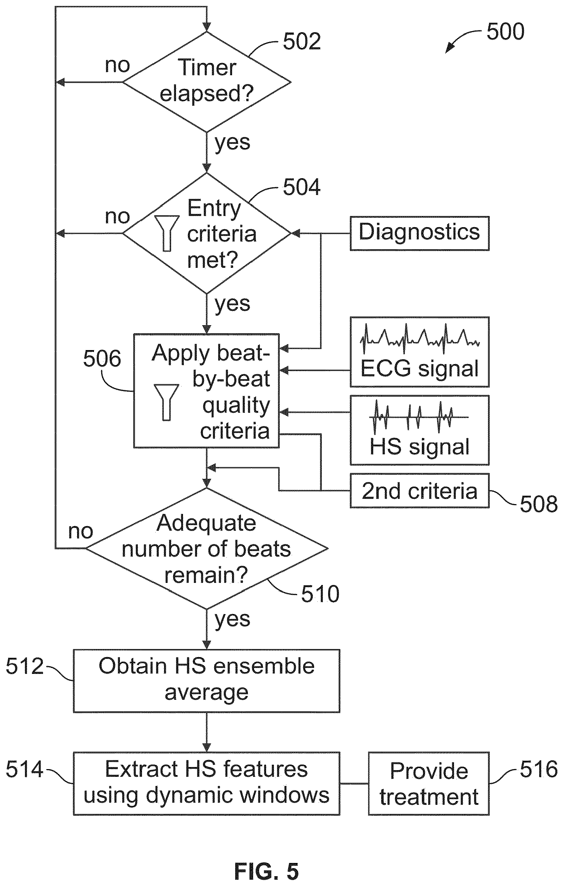

Figure 5 illustrates a process 500 for obtaining a heart sound ensemble for extracting heart sound characteristics for diagnostics and treatment of heart related conditions. In example embodiments any of the systems, devices, IMDs, etc. previously described in Figures 1-4 may be utilized to perform one or more operations of the process 500. Heart sounds may be utilized to diagnose and/or treat heart conditions such as arrhythmias, heart disease, stroke, heart attack, or the like, verify a health condition such as a stroke, heart attack, syncope, etc., and thus treat these heart and health conditions.

The process 500 provides an improved process for obtaining the heart sound signals for analysis by eliminating or removing certain heart sound signals to be analyzed as an ensemble by providing initial criteria based on how the heart sound signals were obtained. In particular, the initial criteria filter the heart sound signals that have a high likelihood of providing false, or inaccurate data or information. For example, the initial criteria can be related to diagnostics from a CA signal such as an ECG, an accelerometer, or the like that can be utilized to determine the activity level of a patient when the heart sound signals are being obtained. As one example the ECG or other biosensors can be utilized to determine the heartrate of the patient during acquisition to determine whether the heart rate is below a tachycardia threshold indicative of no arrythmias and lack of QRS wave oversensing. As another example, posture data from an accelerometer can show if a patient has a static posture such as being supine, having a left-sided recline, having a right sided recline, or the lie. By evaluating how the heart sound data was obtained (e.g., the heart sound acquisition data), certain heart sound signals can be immediately eliminated from additional analysis or consideration to increase the probability that the heart sound signals analyzed provide accurate results. Once this initial acquisition-based filtering occurs, the remaining heart sound signals can be further analyzed, or filtered on a beat-by-beat basis. By providing the initial acquisition-based filtering, the energy consumed as a result of the beat-by-beat analysis is greatly reduced, increasing battery life of the system.

At 502, one or more processors determine whether to collect candidate heart sound signals. In one example the one or more processors determine whether timing control circuitry (e.g., timer) is indicating that a determined period of time has elapsed. In another example, the one or more processors receive data or information that candidate heart sound signals should be collected. For example, the IMD can optionally detect the number of beats per minute of the patient of the heart. When the beats per minute fall below a threshold for a particular patient, an indication may be provided that the patient is sleeping, triggering collection of the candidate heart sound signals when the patient is likely in a known supine position. In another example, a patient may have a routine where they exercise every day at a determined period of time, and then sit and work for the next three to four hours. Thus, a period of a raised heart rate followed by a one-hour period of normal heart rate could trigger the collection of candidate heart sound signals as the patient is known to be exercising, and/or in a sitting position. Still, in one manner or another the one or more processors begin collecting candidate heart sound signals.

At 504, the one or more processors analyze diagnostic characteristics of interest related to the collection of the candidate heart sound signals to determine whether an initial criteria for collection has been met. In particular, the one or more processors analyze data and information (e.g., diagnostic characteristics of interest) provided by other biological sensors, other sensors, or the like to determine whether the candidate heart sound signals being collected are potentially faulty. The candidate heart sound signals themselves are not analyzed during this step, and instead other information and data is analyzed to determine if any conditions may be present that could cause the candidate heart sound signals being collected to have errors or not be reliable for analysis.

In one example, the initial criteria can include or be determined based on diagnostic characteristics of interest obtained from a CA signal such as an ECG, accelerometer, other biosensors, or the like. Such diagnostic characteristics of interest can include activity level obtained from an accelerometer that may be consistently below a determined threshold indicating no movement of a patient (e.g., the last X out of Y beats have activity level below threshold Z). In another example, the one or more processors may obtain or collect a posture measurement or determination from an accelerometer being consistently in a certain orientation indicative of a static subject posture (e.g., the last X posture measurements are supine, left-sided recline, or right-sided recline all indicating potential sleep). In another example, a heart rate sensor may detect that the heart rate of the patient is below a tachycardia threshold indicative of no arrhythmias and a lack of QRS wave oversensing (e.g., the average rate of the last X beats is less than (<) 100 beats-per-minute (bpm)). In another example the initial criteria can be determined based on a combination of diagnostic characteristics of interest that includes one or more of activity, posture, heart rate, or the like. In example embodiments the one or more processors can utilize an algorithm, mathematical formula, mathematical model, decision tree, lookup table, or the like to determine the initial criteria that is based on the diagnostic characteristics of interest. If at 504 the initial criteria have not been met, then any candidate heart sound data, information, signals, etc. obtained while the criteria is not met is automatically discarded.

If at 504, the designated initial criteria are met, then at 506 the one or more processors analyze the remainder candidate heart sound signals to determine whether to discard additional candidate heart sound signals based on a heart sound data quality criteria. In this step, the remainder candidate heart sound signals are analyzed to determine whether the remainder candidate heart sound signals meet the heart sound data quality criteria. Because the initial criteria filter out candidate heart sound signals that have a higher probability of being flawed, the quality criteria do not have to account for catching and eliminating such inherently flawed candidate heart sound signals. Instead, the quality criteria can be significantly fine-tuned with narrower ranges for acceptable data than if the removal of candidate heart sound signals based on the initial criteria was not provided.

In addition, because the quality criteria can in some examples be based on ensemble averages of all of the remainder candidate heart sound signals collected a more accurate filtering of the candidate heart sound signals occurs. For example, if a set of obtained candidate heart sound signals includes S1 data obtained while a patient is exercising and S1 data when the patient is at rest, the average amplitude of the S1 data for such a data set can ultimately end up between the exercise-based candidate heart sound signals and the rest-based candidate heart sound signals. In this manner, if one set has an approximate average amplitude of 10mG while another set has an approximate average amplitude of 5mG, the overall average if the initial filter does not occur would be an amplitude of around 7-8mG. Meanwhile an analysis of the candidate heart sound signals could result in eliminating datapoints greater than +/- 2mG, which would eliminate numerous valid data points. Whereas using a method where diagnostic characteristics of interest are analyzed to eliminate the heart sound data obtained during exercise (or during rest), the average peak amplitude would be in the 5mG range, improving the candidate heart sound signal set that is analyzed for diagnosis, verification, treatment, or the like.

In one example, if the initial criteria are met, the quality criteria can be applied on a stream of remainder candidate CA signals such as ECG and heart sound signals to exclude select beats that do not satisfy the beat-by-beat quality criteria to identify a sub-set of candidate beats to use for HS ensemble representation. The beat-by-beat quality criteria uses information in ECG, heart sound, and device diagnostics to exclude segments of HS waveform that have inadequate quality. Examples of remainder candidate heart sound signals that can be excluded based on the quality criteria can include segments within X time interval of an RR interval smaller than a Y threshold or larger than a Z threshold (e.g., X = 0.5 sec, Y threshold = 0.4 sec and Z threshold = 2 sec). In another example, excluded heart sound data can include segments within X time interval of heart sound signal amplitude > positive out-of-range threshold (Y) or < negative out-of-range threshold (Z) (e.g., X = 0.25 sec, out-of-range Y threshold = 25 mG and out-of-range Z threshold = -50 mG).

In yet another example excluded remainder candidate heart sound signals can include segments within X time interval of ECG signal amplitude > positive out-of-range threshold (Y) or < negative out-of-range threshold (Z) (e.g. X = 0.25 sec, out-of-range Y threshold = 2*median R-wave peak amplitude mV and out-of-range Z threshold = -2*median R-wave peak amplitude mV). In another example the excluded heart sound data may include segments with the heart sound signal > X crossings of Y threshold in Z time interval (e.g., X = 5 crossings of Y threshold = +10 or -15 mG in Z time interval = 0.5 sec).

In another example the excluded remainder candidate heart sound signals can include segments within X time interval of 1^{st} derivative of ECG signal amplitude > positive out-of-range threshold (Y) or < negative out-of-range threshold (Z) (e.g. X = 0.25 sec, out-of-range Y threshold = 12 mV/msec and out-of-range Z threshold = -12 mV/msec). In another example, the excluded remainder heart sound signals may include segments within X time interval of 1^{st} derivative of HS signal amplitude > positive out-of-range threshold (Y) or < negative out-of-range threshold (Z) (e.g. X = 0.25 sec, out-of-range Y threshold = 25 mG/msec and out-of-range Z threshold = -25 mG/msec). As another example, the excluded remainder candidate heart sound signals can include segments within X time interval of device detected PVC beat e.g., X = 1 sec (to exclude beats that would result in aberrant heart sound morphology). In other example, other heart sound quality criteria can be utilized to exclude, or discard segments of the remainder candidate heart sound signals to prevent inaccuracies in determinations.

In one example, after HS signals in the additional candidate HS signals are excluded, the one or more processors generate a final group of final HS signals. The final group of final HS signals are the HS signals that are considered to have the desired fidelity for additional analysis.

Alternatively, after excluding additional candidate heart sound signals at 508, optionally, the one or more processors can analyze the remaining heart sound data to provide auxiliary filtering. In one example the auxiliary filtering can include utilizing morphology criteria on the remaining candidate heart sound signals. For example, the ensemble HS beat representation may be compared to an individual HS morphology on a beat-by-beat basis and if the morphological comparison results in acceptable similarity, then the ensemble HS beat representation is determined to be acceptable. Exemplary comparison methods between ensemble HS beat representation and individual HS beat could be any kind of morphology similarity test such as correlation coefficient. Similar to step 506, once all exclusion of additional candidate heart sound signals is completed to provide the desired fidelity for the HS signals, the final group of HS signals are final HS signals.

After excluding additional heart sound data at 506 and/or 508 to generate the final HS signals, the one or more processors at 510 determine whether the filtered candidate heart sound signals include an adequate, or threshold number of beats that can be analyzed for making additional determinations. In one example, a threshold number of beats such as one-hundred beats are required. In other examples, only fifty beats are required. In yet another example two-hundred beats are required. In yet another example, the threshold number can vary based on historical heartbeat data of the patient, patient conditions, or the like. In another example, the threshold amount can be a percentage of the beats analyzed. So in one example, if at least 50% of the beats analyzed meet both the initial criteria and the heart sound quality criteria, additional analysis can occur. In another example the percentage may be 40%, 60%, etc.

If at 510, an adequate number of beats remain, then at 512 the one or more processors obtain a heart sound ensemble average based on the final HS signals. In particular, the ensemble representation of final HS signals uses a fiducial point on the ECG signal to align in time HS waveforms from consecutive heart beats. In one example the ensemble HS beat representation can be obtained by applying beat-by-beat criteria to one accelerometer axis signal (e.g. Z-axis) or it could be obtained by taking weighted average (or weighted median median) of combination of X, Y or Z axis accelerometer signal after beat-by-beat criteria are applied to these components individually. In one example a determination can be made regarding which axis (e.g., X-axis, Y-axis, Z-axis) is providing the best or strongest HS signals or if a combination of all three axes is providing the best or strongest HS signals. Then, based on this determination, the ensemble averages are obtained accordingly.

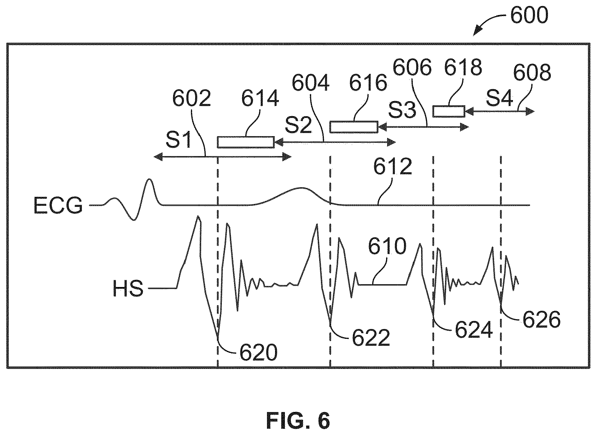

At 512, once the HS ensemble average is obtained, at 514, the one or more processors extract HS features using dynamic time windows. In one example, the graph of Figure 6 informs how such dynamic time windows can be utilized. Figure 6 illustrates a dynamic time window 600 for S1 602, S2 604, S3 606 and S4 608 features of a heart sound wave 610 and an accompanying ECG wave 612. Each of the heart sounds S1-S4 602-608 represent a duration of time during which the related heart sound can be detected. In addition, the graph also illustrates a first blanking period 614, as second blanking period 616, and a third blanking period 618 during which a wave is not analyzed for a heart sound. In particular, once a heart sound is considered detected at a S1 point 620, S2 point 622 or S3 point 624 there is an understanding that S1-S3 points 620-624 (wherein S4 point 626 is also illustrated) are based on a valley that occurs after a peak and that additional waves or fluctuations occur following such peaks and valleys. Consequently, the blanking periods 614-618 are provided to ensure that an echo or left over peak caused by the first HS does not get mistakenly identified as a peak and/or valley resulting from the next heart sound.

An adaptive way can be proposed to identify dynamic time windows (e.g., search windows) of S1, S2, S3 and S4 components of the ensemble HS waveform. In one example, initial bounds of the dynamic time window are defined for S1, S2, S3 and S4 assuming a heart rate of 60 bpm (see figure 6) as follows: 1) S1 dynamic time window starts and ends relative to QRS -wave peak = 0 and 250 msec; 2) post- S1 blanking window of 250 msec relative to S1 peak; 3) S2 dynamic time window starts and ends relative to S1 peak = 250 and 500 msec; 4) post S2 blanking window of 150 msec relative to S2 peak; 5) S3 dynamic time window starts and ends relative to S2 peak = 150 and 300 msec; 6) post S3 blanking window of 50 msec relative to S3 peak; 7) S4 dynamic time window starts and ends relative to S3 peak = 50 and 175 msec; 8) post S4 Blanking window of 125 msec relative to S4 peak. The initial dynamic time window bounds can then be adjusted for heart rate scaling them by median RR interval. In particular, the initial dynamic time window bounds are based on median heart rate of 60 bpm (or RR interval of 1 sec). By multiplying by RR interval, the dynamic time window bounds are scaled to be narrower for faster heart rate (shorter RR interval) or wider for slower heart rate (longer RR interval). Alternatively, dynamic time windows for S1-S4 could be based other ECG features such as onset of QRS, onset/peak of T-wave location or onset/peak of P-wave.

Several direct features and derived features are extracted for trending and potential predictive analytics. These features can include QRS peak amplitude and peak location for the ECG wave. In addition, ECG features can also include T wave peak amplitude and peak location. HS features can include S1, S2, S3, S4 peak absolute amplitude and peak timings, S1, S2, S3, S4 onset/offset timing, S1, S2, S3, S4 waveforms absolute integral (or average) between search window bounds, or the like. In addition, several derived features may be calculated for trending and potential predictive analytics for the HS including QRS-peak to S1, S2, S3, S4 intervals, S1 to S2 interval (surrogate for systolic ejection interval), absolute S2-S1 peak amplitude, or the like. In one example embodiment, the operation of obtaining ensemble representation and trending could be all or partially be done on device or alternatively signal segments stored by device could be transmitted to data cloud for further processing and trending. In this manner improved HS signal acquisition and analysis is provided.

With reference back to Figure 5, once the HS features are extracted using dynamic time windows at 514, at 516, the one or more processors treat the patient by controlling the IMD based on the ensemble candidate HS signals. The improved ensemble candidate HS signals are utilized for therapy purposes, improving therapy for a patient.

### Closing Statements

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method, or computer (device) program product. Accordingly, aspects may take the form of an entirely hardware embodiment or an embodiment including hardware and software that may all be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random-access memory (RAM), a dynamic random-access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for conducting operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for conducting the operations and provide this code through its network interface via a network to a second device having a second processor for execution of the code on the second device.

Aspects are described herein with reference to the Figures, which illustrate example methods, devices, and program products according to various example embodiments. These program instructions may be provided to a processor of a general-purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only and are thus not intended to limit in any way the definition and/or meaning of the term "controller." The units/modules/applications herein may execute a set of instructions that are stored in one or more storage elements, in order to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being conducted in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

## Claims

1. An implantable medical device, IMD, (100) comprising:
a monitoring device (244) configured to obtain cardiac activity signals;
an accelerometer (235) configured to obtain candidate heart sound (HS) signals;
a memory (260) configured to store program instructions;
one or more processors (220) configured, when executing the program instructions, to:
analyze the cardiac activity signals based on initial criteria;
generate a HS ensemble; and
control the IMD (100) based on the HS ensemble, **characterized in that** the one or more processors (220) are configured, when executing the program instructions, to:
eliminate candidate HS signals based on the analyzing to provide remainder candidate HS signals;
analyze the remainder candidate HS signals based on HS quality criteria based on HS characteristics of interest and related to the remainder candidate HS signals;
eliminate additional candidate HS signals from the remainder candidate HS signals to provide final HS signals; and
generate the HS ensemble based on the final HS signals.

2. The IMD of claim 1, wherein the one or more processors (220) are further configured, when executing the program instructions, to:
determine HS characteristics of interest based on the HS ensemble;
analyze the HS characteristics of interest with a dynamic time window; and
vary the dynamic time window based on the analyzing of the HS characteristics of interest.

3. The IMD of claim 2, wherein the dynamic time window includes a blanking window.

4. The IMD of claim 2 or 3, wherein the dynamic window includes initial search window bounds that are adjusted based on the HS characteristics of interest.

5. The IMD of any one of claims 1 to 4, wherein the one or more processors (220) are configured, when executing the program instructions, to analyze a diagnostic characteristic of interest related to the cardiac activity signals.

6. The IMD of claim 5, wherein the diagnostic characteristic of interest is at least one of RR interval, QRS-wave amplitude, R-wave stability, or heart beat rhythm.

7. The IMD of any one of claims 1 to 6, wherein the cardiac activity signals are ECG signals.

8. The IMD of any one of claims 1 to 7, wherein the initial criteria include at least one of posture or activity level.

9. The IMD of any one of claims 1 to 8, wherein the HS quality criteria are based on HS characteristics of interest that include at least one of HS amplitude or HS level crossing.

10. The IMD of any one of claims 1 to 9, wherein the one or more processors (220) are further configured, when executing the program instructions, to determine whether the HS ensemble has a threshold number of beats.

11. The IMD of any one of claims 1 to 10, wherein the HS quality criteria are first HS quality criteria, the one or more processors (220) further configured, when executing the program instructions, to:
analyze the remainder candidate HS signals based on second HS quality criteria; and
eliminate the additional candidate HS signals from the remainder candidate HS signals based on the first HS quality criteria and the second HS quality criteria to provide the final group of the final HS signals.

12. The IMD of claim 11, wherein the first HS quality criteria are based on HS characteristics of interest that include at least one of HS amplitude or HS level crossing, and the second HS quality criteria are based on HS morphology.

13. The IMD of any one of claims 1 to 12, wherein the one or more processors (220) are configured, when executing the program instructions, to:
determine diagnostic characteristics of interest contemporaneously with the candidate HS signals;
analyze the diagnostic characteristics of interest based on initial criteria;
determine whether a threshold number of beats is within the HS ensemble; and
control the IMD (100) based on the HS ensemble when the threshold number of beats are within the HS ensemble.

14. The IMD of claim 13, wherein the one or more processors (220) are configured, when executing the program instructions, to
determine the diagnostic characteristics of interest are based on the cardiac activity signals.

## Patentansprüche

1. Implantierbare medizinische Vorrichtung, IMD, (100), umfassend:
eine Überwachungsvorrichtung (244), die dazu konfiguriert ist, Herzaktivitätssignale zu erlangen;
einen Beschleunigungsmesser (235), der dazu konfiguriert ist, Kandidatenherzton(HS)-Signale zu erlangen;
einen Speicher (260), der dazu konfiguriert ist, Programmanweisungen zu speichern;
einen oder mehrere Prozessoren (220), die dazu konfiguriert sind, beim Ausführen der Programmanweisungen Folgendes zu tun: Analysieren der Herzaktivitätssignale basierend auf anfänglichen Kriterien;
Erzeugen eines HS-Ensembles; und
Steuern der IMD (100) basierend auf dem HS-Ensemble, **dadurch gekennzeichnet, dass** der eine oder die mehreren Prozessoren (220) dazu konfiguriert sind, beim Ausführen der Programmanweisungen Folgendes zu tun:
Eliminieren von Kandidaten-HS-Signalen basierend auf dem Analysieren, um Restkandidaten-HS-Signale bereitzustellen;
Analysieren der Restkandidaten-HS-Signale basierend auf HS-Qualitätskriterien basierend auf HS-Eigenschaften von Interesse und in Bezug auf die Restkandidaten-HS-Signale;
Eliminieren zusätzlicher Kandidaten-HS-Signale aus den Restkandidaten-HS-Signalen, um endgültige HS-Signale bereitzustellen; und
Erzeugen des HS-Ensembles basierend auf den finalen HS-Signalen.

2. IMD nach Anspruch 1, wobei der eine oder die mehreren Prozessoren (220) ferner dazu konfiguriert sind, beim Ausführen der Programmanweisungen Folgendes zu tun:
Bestimmen von HS-Eigenschaften von Interesse basierend auf dem HS-Ensemble;
Analysieren der HS-Eigenschaften von Interesse mit einem dynamischen Zeitfenster; und
Variieren des dynamischen Zeitfensters basierend auf dem Analysieren der HS-Eigenschaften von Interesse.

3. IMD nach Anspruch 2, wobei das dynamische Zeitfenster ein Austastfenster beinhaltet.

4. IMD nach Anspruch 2 oder 3, wobei das dynamische Fenster anfängliche Suchfenstergrenzen beinhaltet, die basierend auf den HS-Eigenschaften von Interesse angepasst werden.

5. IMD nach einem der Ansprüche 1 bis 4, wobei der eine oder die mehreren Prozessoren (220) dazu konfiguriert sind, beim Ausführen der Programmanweisungen eine diagnostische Eigenschaft von Interesse in Bezug auf die Herzaktivitätssignale zu analysieren.

6. IMD nach Anspruch 5, wobei die diagnostische Eigenschaft von Interesse mindestens eines von RR-Intervall, QRS-Wellenamplitude, R-Wellenstabilität oder Herzschlagrhythmus ist.

7. IMD nach einem der Ansprüche 1 bis 6, wobei die Herzaktivitätssignale EKG-Signale sind.

8. IMD nach einem der Ansprüche 1 bis 7, wobei die anfänglichen Kriterien mindestens eines von Körperhaltung oder Aktivitätsniveau beinhalten.

9. IMD nach einem der Ansprüche 1 bis 8, wobei die HS-Qualitätskriterien auf HS-Eigenschaften von Interesse basieren, die mindestens eines von HS-Amplitude oder HS-Niveauübergängen beinhalten.

10. IMD nach einem der Ansprüche 1 bis 9, wobei der eine oder die mehreren Prozessoren (220) ferner dazu konfiguriert sind, beim Ausführen der Programmanweisungen zu bestimmen, ob das HS-Ensemble eine Schwellenwertanzahl von Schlägen aufweist.

11. IMD nach einem der Ansprüche 1 bis 10, wobei die HS-Qualitätskriterien erste HS-Qualitätskriterien sind, wobei der eine oder die mehreren Prozessoren (220) ferner dazu konfiguriert sind, beim Ausführen der Programmanweisungen, Folgendes zu tun:
Analysieren der Restkandidaten-HS-Signale basierend auf zweiten HS-Qualitätskriterien; und
Eliminieren der zusätzlichen Kandidaten-HS-Signale aus den Restkandidaten-HS-Signalen basierend auf den ersten HS-Qualitätskriterien und den zweiten HS-Qualitätskriterien, um die endgültige Gruppe der endgültigen HS-Signale bereitzustellen.

12. IMD nach Anspruch 11, wobei die ersten HS-Qualitätskriterien auf HS-Eigenschaften von Interesse basieren, die mindestens eines von HS-Amplitude oder HS-Niveauübergängen beinhalten, und die zweiten HS-Qualitätskriterien auf HS-Morphologie basieren.

13. IMD nach einem der Ansprüche 1 bis 12, wobei der eine oder die mehreren Prozessoren (220) dazu konfiguriert sind, beim Ausführen der Programmanweisungen Folgendes zu tun:
Bestimmen diagnostischer Eigenschaften von Interesse gleichzeitig mit den Kandidaten-HS-Signalen;
Analysieren der diagnostischen Eigenschaften von Interesse basierend auf anfänglichen Kriterien;
Bestimmen, ob eine Schwellenwertanzahl von Schlägen innerhalb des HS-Ensembles liegt; und
Steuern der IMD (100) basierend auf dem HS-Ensemble, wenn die Schwellenwertanzahl von Schlägen innerhalb des HS-Ensembles liegt.

14. IMD nach Anspruch 13, wobei der eine oder die mehreren Prozessoren (220) dazu konfiguriert sind, beim Ausführen der Programmanweisungen Folgendes zu tun:
Bestimmen, dass die diagnostischen Eigenschaften von Interesse auf den Herzaktivitätssignalen basieren.

## Revendications

1. Dispositif médical implantable, IMD, (100) comprenant :
un dispositif de surveillance (244) configuré pour obtenir des signaux d'activité cardiaque ;
un accéléromètre (235) configuré pour obtenir des signaux de bruits cardiaques, HS, candidats ;
une mémoire (260) configurée pour stocker des instructions de programme ;
un ou plusieurs processeurs (220) configurés, lors de l'exécution des instructions de programme, pour :
analyser les signaux d'activité cardiaque sur la base de critères initiaux ;
générer un ensemble de HS ; et
commander l'IMD (100) sur la base de l'ensemble de HS, **caractérisé en ce que** lesdits un ou plusieurs processeurs (220) soient configurés, lors de l'exécution des instructions de programme, pour :
éliminer des signaux de HS candidats sur la base de l'analyse afin de fournir des signaux de HS candidats restants ;
analyser les signaux de HS candidats restants sur la base de critères de qualité de HS qui sont basés sur des caractéristiques de HS d'intérêt et qui sont liés aux signaux de HS candidats restants ;
éliminer des signaux de HS candidats supplémentaires des signaux de HS candidats restants afin de fournir des signaux de HS finaux ; et
générer l'ensemble de HS sur la base des signaux de HS finaux.

2. IMD selon la revendication 1, dans lequel lesdits un ou plusieurs processeurs (220) sont en outre configurés, lors de l'exécution des instructions de programme, pour :
déterminer des caractéristiques de HS d'intérêt sur la base de l'ensemble de HS ;
analyser les caractéristiques de HS d'intérêt avec une fenêtre temporelle dynamique ; et
faire varier la fenêtre temporelle dynamique sur la base de l'analyse des caractéristiques de HS d'intérêt.

3. IMD selon la revendication 2, dans lequel la fenêtre temporelle dynamique comprend une fenêtre de masquage.

4. IMD selon la revendication 2 ou 3, dans lequel la fenêtre dynamique comprend des limites initiales de la fenêtre de recherche qui sont ajustées sur la base des caractéristiques de HS d'intérêt.

5. IMD selon l'une quelconque des revendications 1 à 4, dans lequel lesdits un ou plusieurs processeurs (220) sont configurés, lors de l'exécution des instructions de programme, pour analyser une caractéristique diagnostique d'intérêt liée aux signaux d'activité cardiaque.

6. IMD selon la revendication 5, dans lequel la caractéristique diagnostique d'intérêt est au moins un parmi un intervalle RR, une amplitude d'onde QRS, une stabilité d'onde R ou un rythme cardiaque.

7. IMD selon l'une quelconque des revendications 1 à 6, dans lequel les signaux d'activité cardiaque sont des signaux ECG.

8. IMD selon l'une quelconque des revendications 1 à 7, dans lequel le critère initial comprend au moins un élément parmi la posture ou le niveau d'activité.

9. IMD selon l'une quelconque des revendications 1 à 8, dans lequel les critères de qualité de HS sont basés sur des caractéristiques de HS d'intérêt qui comprennent au moins un élément parmi une amplitude de HS ou un franchissement de niveau de HS.

10. IMD selon l'une quelconque des revendications 1 à 9, dans lequel lesdits un ou plusieurs processeurs (220) sont en outre configurés, lors de l'exécution des instructions de programme, pour déterminer si l'ensemble de HS atteint un nombre seuil de battements.

11. IMD selon l'une quelconque des revendications 1 à 10, dans lequel les critères de qualité de HS sont des premiers critères de qualité de HS, lesdits un ou plusieurs processeurs (220) étant en outre configurés, lors de l'exécution des instructions de programme, pour :
analyser les signaux de HS candidats restants sur la base de seconds critères de qualité de HS ; et
éliminer les signaux de HS candidats supplémentaires des signaux de HS candidats restants sur la base des premiers critères de qualité de HS et des seconds critères de qualité de HS afin de fournir le groupe final des signaux de HS finaux.

12. IMD selon la revendication 11, dans lequel les premiers critères de qualité de HS sont basés sur des caractéristiques de HS d'intérêt qui comprennent au moins un élément parmi une amplitude de HS ou un franchissement de niveau de HS, et les seconds critères de qualité de HS sont basés sur une morphologie de HS.

13. IMD selon l'une quelconque des revendications 1 à 12, dans lequel lesdits un ou plusieurs processeurs (220) sont configurés, lors de l'exécution des instructions de programme, pour :
déterminer des caractéristiques diagnostiques d'intérêt simultanément aux signaux de HS candidats ;
analyser les caractéristiques diagnostiques d'intérêt sur la base de critères initiaux ;
déterminer si un nombre seuil de battements est présent dans l'ensemble de HS ; et
commander l'IMD (100) sur la base de l'ensemble de HS lorsque le nombre seuil de battements est présent dans l'ensemble de HS.

14. IMD selon la revendication 13, dans lequel lesdits un ou plusieurs processeurs (220) sont configurés, lors de l'exécution des instructions de programme, pour :
déterminer les caractéristiques diagnostiques d'intérêt sur la base des signaux d'activité cardiaque.
